# EUROPEAN PATENT APPLICATION

(11) **EP 0 893 422 A1**
(43) Date of publication of application: **27.01.1999**
(21) Application number: 98112990.1
(22) Date of filing: 13.07.1998
(51) Int. Cl.: C07C 31/125, C12P 41/00

(54) **Optically active alcohol and process for the production thereof**

(30) Priority: 18.07.1997 JP 194112/97
(71) Applicant: MITSUBISHI GAS CHEMICAL COMPANY, INC., Tokyo 100-0005 (JP)
(72) Inventor: Mine, Takakiyo, 22, Wadai, Tsukuba-shi, Ibaraki-ken (JP); Yui, Tomoyuki, 22, Wadai, Tsukuba-shi, Ibaraki-ken (JP)
(74) Representative: Kraus, Walter, Dr.

(57) **Abstract**

An R-configuration or S-configuration optically active alcohol of the general formula (1),

CH₃C*H(OH)(CH₂)ₘCH(CₙH₂ₙ₊₁)₂ (1)

wherein C* is an asymmetric carbon atom, m is an integer of 0 to 3, and n is an integer of 1 to 3; and a process for the production thereof, which comprises asymmetrically trans-esterifying a corresponding racemic alcohol to conduct the optical resolution. This is preferably carried out in the presence of a lipase extracted from Candida antarcia as catalyst.

## Description

### Detailed Description of the Invention

### Field of the Invention

The present invention relates to a novel optically active secondary alcohol having a methyl group on an asymmetric carbon atom and branched alkyl chains at its terminal, the branched alkyl chains having the same number of carbon atoms; and a process for the production thereof.

### Prior Art

While optically active compounds have been used in the fields of medicaments and agricultural chemicals, they have been recently attracting attention as functional materials for ferroelectric liquid crystal and organic non-linear materials. For example, in the field of organic non-linear materials, molecules of organic materials preferably have an asymmetric center for producing secondary non-linear optical effect (e.g., Yamaguchi, Nakano and Fueno, "Kagaku (Chemistry)" 42 (11), 757 (1987)). In the field of ferroelectric liquid crystal compounds, liquid crystal compounds are required to have an optically active structure so that a liquid crystal can exhibit a ferroelectricity (e.g., Johno, Fukuda, Journal of Organic Synthesis Chemistry Society, 47 (6), 568 (1989)).

In recent years, further, anti-ferroelectric liquid crystal compounds are attracting considerable attention, but the anti-ferroelectric liquid crystal compounds are required to have an optically active structure, like ferroelectric liquid crystal compounds. In the above fields, optically active 2-butanol, 2-octanol, 2-methyl-1-butanol, an amino acid derivative or the like has been used as an optically active source. However, obtained optically active materials are limited in characteristics so long as the above optically active compounds are used.

In the field of ferroelectric liquid crystals, recently, attempts have been vigorously made to synthesize ferroelectric liquid crystals from the following optically active alcohols in which a perfluoroalkyl group is substituted on their asymmetric carbon atom (C*)(see, e.g., JP,A 64-3154, JP,A 1-316339, JP,A 1-316367, JP,A 1-316372, JP,A 2-225434 and JP,A 2-229128).
(1) CF₃C*H(OH)CH₂COOC₂H₅
(2) CF₃C*H(OH)CH₂CH₂OC₂H₅
(3) CF₃C*H(OH)CH₂CH₂CH₂OC₂H₅
(4) CF₃C*H(OH)CH₂CH₂CH₂CH₂OC₂H₅
(5) CF₃C*H(OH)C₆H₁₃
(6) CF₃C*H(OH)C₈H₁₇
(7) C₂F₅C*H(OH)C₈H₁₇

Ferroelectric liquid crystal compounds derived from the above alcohols all give high spontaneous polarization and give also relatively fast response speed since a perfluoroalkyl group having high electro-negativity is substituted on the asymmetric carbon atom of each. It is also known that a liquid crystal compound derived from the above (5), (6) or (7) easily gives a liquid crystal compound having an anti-ferroelectric phase, and hence, these alcohols are attracting attention as particularly characteristic optically active alcohol.

Further, with regard to the process for the synthesis of an optically active alcohol of CF₃C*H(OH)(CH₂)ₘOCₙH₂ₙ₊₁ (in which m is an integer of 2 to 7 and n is an integer of 1 to 4), the present inventors made detailed studies on the process for the synthesis of an intended optically active alcohol having a high optical purity without using an expensive raw material, ethyl trifluoroacetoacetate, and on liquid crystal compounds derived therefrom, and it was found that the above alcohol gives very useful anti-ferroelectric liquid crystal compounds or ferrielectric liquid crystal compounds (see, JP,A 5-65486 and JP,A 8-33555).

When an anti-ferroelectric liquid crystal compound or a ferrielectric liquid crystal compound is derived from an optically active alcohol containing an asymmetric carbon atom having a fluoroalkyl group substituted thereon, however, the resulting liquid crystal compound shows very high spontaneous polarization. When the spontaneous polarization is large, the response speed is fast, and the high spontaneous polarization is advantageous in this respect. With an increase in the spontaneous polarization, however, the interaction of an insulating film and an aligned film with the liquid crystal compound in a cell increases, and as a result, the deformation of hysteresis of electro-optical response increases to an extraordinary extent. There is therefore liable to be caused a problem that no drive margin is permitted.

There is therefore desired a liquid crystal compound which shows a small spontaneous polarization and, on the other hand, which is free of problems on a response speed and a tilt angle.

An optically active secondary alcohol can be produced by various methods.

In view of economic performance, however, it is not expedient to use an optically active compound as a raw material, since it is expensive.

On the other hand, an optically active alcohol may be also produced by asymmetric synthesis. For example, it is thinkable to employ a method in which an optically active alcohol is produced by preparing a corresponding ketone compound as a precursor and then, asymmetrically reducing it in the presence of an asymmetrically reducing catalyst. In this case, however, the asymmetrically reducing catalyst is very expensive. Further, a product having a high optical purity cannot be always obtained, and only either an R-configuration compound or an S-configuration compound is obtained.

For example, there is known a method in which a prochiral ketone is asymmetrically reduced in the presence of a catalyst which is a complex in which (1R,2R)-1,2-diphenyl-2-aminoethanol contains a boron atom as a ligand (see, J. Yaozhong, et al., Tetrahedron: Asymmetry, 5(7), 1211 (1994)).

The above method is remarkably effective for aromatic ketones. However, it cannot be said to be effective for aliphatic ketones since the obtained enantiomers have a very low optical purity.

In another method, it is thinkable to asymmetrically hydrolyze a proper ester which is a precursor for an optically active compound, such as an acetate. An enzyme can be used as an effective asymmetric hydrolysis agent. The asymmetric hydrolysis of an acetate with lipase has been proposed by Kitazume et al (see, T. Kitazume et al., J. Org. 52, 3211 (1987), JP-A-2-282340).

According to Kitazume et al, the acetate of the formula, CF₃CH(OCOCH₃)CₙH₂ₙ₊₁ is asymmetrically hydrolyzed in the presence of lipase MY in a phosphate buffer solution.

However, the capability of lipase MY for recognizing asymmetry is greatly dependent upon the chemical structure of a compound to be hydrolyzed. And, the optical purity of obtained hydrolysis products greatly varies from 55 to 98 ee% depending upon chemical structures as shown in Table 1 in the above literature by Kitazume et al.

The above results show that it is difficult to evaluate whether or not an intended compound can be effectively asymmetrically hydrolyzed and that it is found only after a reaction whether or not an intended alcohol having a high optical purity can be obtained.

Further, there is another serious problem that the capability for asymmetry recognition is not at all exhibited when some substituent is on an asymmetric carbon atom.

For example, the lipase MY exhibits the remarkably high capability for asymmetry recognition in the asymmetric hydrolysis of CF₃C*H(OCOCH₃)(CH₂)₅OC₂H₅. However, the lipase MY does not show any asymmetry recognition for an ester of a secondary alcohol, CH₃C*H(OCOCH₃)C₆H₁₃, in which a methyl group is substituted on the asymmetric carbon atom.

In addition, a secondary alcohol is also produced by a method in which a secondary racemic alcohol is asymmetrically trans-esterified in the presence of a proper enzyme to carry out the optical resolution thereof.

One example is a reaction of asymmetric trans-esterification in the presence of a lipase (derived from porcine pancreas) in an organic solvent (see, A. M. Klibanov, et al., J. Am. Chem. Soc. 1985, 106, 7072).

However, no lipase having high activity and high enantio-selectivity has been known. The asymmetric hydrolysis or the optical resolution by asymmetric trans-esterification using an enzyme is advantageous in that both R-configuration and S-configuration optically active alcohols are easily obtained.

### Problems to be Solved by the Invention

The present invention has been made in view of the above situation, and it is an object of the present invention to provide a novel optically active secondary alcohol having a methyl group on an asymmetric carbon atom and having branched alkyl chains at the terminal, the branched alkyl chains having the same number of carbon atoms; and a process for the advantageous production thereof.

### Means to Solved the Problems

According to studies by the present inventors, it has been found that the above object of the present invention is achieved by an R-configuration or S-configuration optically active alcohol of the general formula (1),

CH₃C*H(OH)(CH₂)ₘCH(CₙH₂ₙ₊₁)₂ (1)

wherein C* is an asymmetric carbon atom, m is an integer of 0 to 3, and n is an integer of 1 to 3.

The optically active alcohol of the above general formula (1) has structural features that a methyl group is substituted on an asymmetric carbon atom (C*), that branched alkyl groups -CH(CₙH₂ₙ₊₁)₂ are present at the terminal, and that two -CₙH₂ₙ₊₁ in the branched alkyl group are alkyl groups having the same numbers of carbon atoms. In the above general formula (1), m is an integer of 0 to 3, and n is an integer of 1 to 3, preferably 1 or 2.

According to studies by the present inventors, further, an industrially advantageous process for the production of the optically active alcohol of the above general formula (1) has been found. That is, according to the present invention, there is provided a process for the production of an optically active alcohol by optical resolution of a racemic alcohol of the general formula (2),

CH₃CH(OH)(CH₂)ₘCH(CₙH₂ₙ₊₁)₂ (2)

wherein m is an integer of 0 to 3 and n is an integer of 1 to 3,
characterized in that the optical resolution is carried out by asymmetrically trans-esterifying the said racemic alcohol.

In the present invention, vinyl propionate is suitable as an esterification agent for the above asymmetric trans-esterification.

In the present invention, it is preferred to use lipase derived from Candida antarcia microorganism as a catalyst for the above asymmetric trans-esterification. As the above lipase, an immobilized enzyme prepared by immobilizing the lipase on porous acrylic resin particles can be suitably used. The immobilized enzyme is commercially available from Novo Nordisk A/S, and it can be used as it is.

The lipase derived from Candida antarcia, used in the present invention, has remarkably high reactivity per unit amount, has high enantio selectivity, i.e., the selectivity to convert R-configuration to a propionate ester selectively, and has higher reactivity as compared with lipase derived from porcine pancreas and lipase derived from Pseudomomas which are known to have capability of the optical resolution of a secondary alcohol. Even the small amount thereof can give high reactivity.

The reaction rate is in proportion to an amount of lipase used, and the amount thereof is therefore determined depending upon a predetermined reaction time. Generally, however, the amount of the above lipase per mole of the racemic alcohol as a precursor is preferably in the range of from 0.1 to 10 g.

The reaction temperature for the above asymmetric trans-esterification is preferably between 20°C and 40°C for attaining a sufficient reaction rate and a sufficient enantio selectivity.

### Effect of the Invention

The present invention can provide a novel optically active secondary alcohol having a methyl group on an asymmetric carbon atom and having branched alkyl chains having the same number of carbon atoms at its terminal, at a high optical purity, and an economical and simple process for the production thereof.

### Examples

The present invention will be explained in detail with reference to Examples hereinafter, while the present invention shall not be limited thereto.

### Example 1 Preparation of R-(-)-5-methylhexan-2-ol (formula (1): m = 2, n = 1 (E1))

### (1) Preparation of 5-methylhexan-2-ol (racemic compound)

40 Grams of a 12 % NaBH₄ solution (NaOH aqueous solution) was gradually added dropwise to 40 g of commercially available 5-methyl-2-hexanone. The mixture was stirred at room temperature for 3 hours, then, water was added, and the mixture was extracted with ether. An ether layer was washed with 6N hydrochloric acid, and then washed with water until the ether layer almost showed neutrality, and the resultant ether layer was further washed with a saturated sodium chloride aqueous solution.

The above-washed ether layer was dried over anhydrous sodium sulfate, and the ether was distilled off to give 35 g of a crude product. The crude product was purified by distillation, to give an end product (30 Torr, 74°C, yield 70 %).

### (2) Preparation of R-(-)-5-methylhexane-2-propionate

13 Grams of vinyl propionate and 300 mg of lipase (Novozym 435) immobilized on porous acrylic resin particles were added to 25 g of the secondary alcohol obtained in (1), and the mixture was stirred at room temperature for 24 hours.

After completion of the reaction, the lipase was filtered off, and the remainder was washed with hexane. Then, the hexane, alcohol as a raw material, etc., were distilled off.

The above-obtained product was purified by a silica gel column chromatography to give an oily end product (22 Torr, 84°C: yield 65 %) and S-(+)-5-methylhexan-2-ol (yield 80 %).

### (3) Preparation of R-(-)-5-methylhexan-2-ol

11 Grams of the R-(-)-5-methylhexan-2-propionate obtained in (2) was added to a solution of 7 g of potassium hydroxide in 20 ml of water/methanol (1/3), and the mixture was stirred at room temperature for 1 hour. After completion of the reaction, the reaction mixture was extracted with ether, an organic layer was washed with water and with a saturated sodium chloride aqueous solution, and the washed mixture was dried over anhydrous sodium sulfate. The anhydrous sodium sulfate was filtered off, and then the ether was distilled off to give an end product (20 Torr, 66°C, yield 88 %).

### Example 2 Preparation of R-(-)-6-methylhetpan-2-ol (formula (1): m = 3, n = 1, (E2))

An end product was obtained from commercially available 6-methylhetpan-2-ol by carrying out the same optical resolution as that in Example 1.

### Example 3 Preparation of R-(-)-3-methylbutan-2-ol (formula (1): m = 0, n = 1, (E3))

A mixture of R-(-)-3-methylbutan-2-propionate and S-(+)-3-methylbutan-2-ol was obtained from commercially available 3-methyl-2-butanol in the same manner as in the (2) of Example 1.

The above mixture was dissolved in ether, octanoic acid chloride was added, pyridine was dropwise added, and the mixture was stirred at room temperature for 30 hours. To the resultant mixture was added 6N hydrochloric acid, the mixture was extracted with ether, and an ether layer was washed with water.

The washed ether layer was dried over anhydrous sodium sulfate, then, the solvent was distilled off, and then R-(-)-3-methylbutane-2-propionate and S-(+)-3-methylbutan-2-octanoate were separated from each other by distillation under reduced pressure.

The above R-(-)-3-methylbutane-2-propionate was treated in the same manner as in the (3) of Example 1, to give an end product.

### Example 4 Preparation of R-(-)-4-methylpentan-2-ol (formula (1): m = 1, n = 1, (E4))

An end product was obtained from commercially available 4-methylpentan-2-ol by carrying out the same optical resolution as that in Example 3.

### Example 5 Preparation of R-(-)-5-ethylheptan-2-ol (formula (1): m = 2, n = 2, (E5))

### (1) Preparation of 5-ethylheptan-2-ol (racemic compound)

4.4 Grams of sodium was dissolved in a solution mixture containing 26 mol of methyl acetate and 60 ml of absolute ethanol, and the mixture was cooled to room temperature. To the mixture was dropwise added 0.17 mol of 3-ethyl-1-butyl bromide with stirring, and the resultant mixture was refluxed under heating for 50 hours.

The obtained reaction mixture was filtered by means of suction, then, the ethanol was distilled off, and a residue was dissolved in 80 ml of a 10 % sodium hydroxide aqueous solution. The resultant solution was stirred at room temperature for 24 hours, and then acidified with concentrated hydrochloric acid. The solution was cooled and then extracted with ether, to separate an organic layer.

To the organic layer was added 30 g of a 12 % NaBH₄ solution (NaOH aqueous solution) over 2 hours. The mixture was stirred at room temperature for 2 hours, water was added, and the mixture was extracted with ether. An ether layer was washed with 6N hydrochloric acid, and then washed with water until the ether layer almost showed neutrality. Further, the ether layer was washed with a saturated sodium chloride aqueous solution. The ether layer was dried over anhydrous sodium sulfate, and the ether was distilled off under atmospheric pressure, to give a crude product. The crude product was purified by distillation under reduced pressure (30 Torr, 103°C) to give a racemic alcohol as an end product (0.040 mol, yield 23 %).

The above-obtained racemic alcohol was treated in the same manner as in the (2) and (3) of Example 1, to give an optically active alcohol as an end product.

### Example 6 Preparation of R-(-)-4-ethylhexan-2-ol (formula (1): m = 1, n = 2, (E6))

### (1) Preparation of 4-ethylhexan-2-ol (racemic compound)

0.270 Mole of magnesium metal was placed in a round-bottom flask, the atmosphere in the flask was replaced with nitrogen, and then 100 ml of dry tetrahydrofuran was added. To this was added a solution of 0.110 mol of 1-bromo-2-ethylbutane in 100 ml of dry tetrahydrofuran so as to adjust a reaction temperature to 40°C or lower. The reaction mixture was allowed to stand for 1 hour, and then 0.280 mol of acetaldehyde (tetrahydrofuran solution having a concentration of 35 %) was dropwise added so as to adjust the reaction temperature to 50°C or lower. Then, the mixture was allowed to react at 50°C for 2 hours.

After completion of the reaction, 150 ml of 6N hydrochloric acid was dropwise added, then, the reaction mixture was stirred at room temperature for 2 hours, and an organic layer was separated. The organic layer was washed with water until it almost showed neutrality, and further, it was washed with a saturated sodium chloride aqueous solution. The organic layer was dried over anhydrous sodium sulfate, the tetrahydrofuran was distilled off under reduced pressure to give a crude product. The crude product was purified by distillation under reduced pressure (20 Torr, 75°C) to give a racemic alcohol as an end product.

The above-obtained racemic alcohol was treated in the same manner as in the (2) and (3) of Example 1 to give an optically active alcohol as an end product.

Table 1 shows NMR spectrum data of the end products (E1 to E6) obtained in Examples 1 to 6.

Further, the end products (E1 to E6) obtained in Examples 1 to 6 were each measured for optical purity by the following method.

The obtained optically active alcohols were converted to acetates with pyridine/acetic anhydride. The obtained acetates were analyzed with a gas chromatograph (CP Cyclodex β236M) for the analysis of optically active compounds, and each optically active alcohol was determined for a purity on the basis of peak area ratio of two enantiomers. Further, each optically active alcohol was measured for a specific rotation.

Table 2 shows the results.

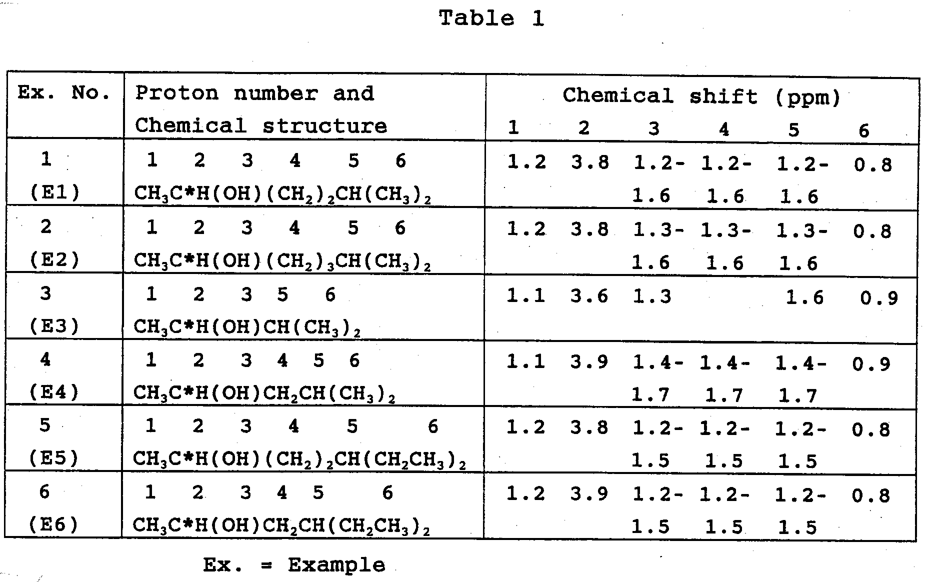

**Table 2**

| Ex. No. | Chemical structure | Optical purity (%ee) | Specific rotation*1 (°) |
|---|---|---|---|
| 1 (E1) | CH₃C*H(OH)(CH₂)₂CH(CH₃)₂ | 97.3 | -9.6 |
| 2 (E2) | CH₃C*H(OH)(CH₂)₃CH(CH₃)₂ | 97.8 | -9.2 |
| 3 (E3) | CH₃C*H(OH)CH(CH₃)₂ | 92.9 | -2.3 |
| 4 (E4) | CH₃C*H(OH)CH₂CH(CH₃)₂ | 95.8 | -18.6 |
| 5 (E5) | CH₃C*H(OH)(CH₂)₂CH(CH₂CH₃)₂ | 97.7 | -6.1 |
| 6 (E6) | CH₃C*H(OH)CH₂CH(CH₂CH₃)₂ | 95.1 | -16.2 |

| | | | |
|---|---|---|---|
| *1: Measured with sodium D ray, at 29° C. | | | |

## Claims

1. An R-configuration or S-configuration optically active alcohol of the general formula (1),
CH₃C*H(OH)(CH₂)ₘCH(CₙH₂ₙ₊₁)₂ (1)
wherein C* is an asymmetric carbon atom, m is an integer of 0 to 3, and n is an integer of 1 to 3.

2. The optically active alcohol of claim 1, wherein n in the general formula (1) is 1 or 2.

3. The optically active alcohol of claim 1, wherein the optically active alcohol has an R-configuration.

4. The optically active alcohol of claim 1, wherein the optically active alcohol has an S-configuration.

5. A process for the production of an opticaily active alcohol by optical resolution of a racemic alcohol of the general formula (2),
CH₃CH(OH)(CH₂)ₘCH(CₙH₂ₙ₊₁)₂ (2)
wherein m is an integer of 0 to 3 and n is an integer of 1 to 3,
characterized in that the optical resolution is carried out by asymmetrically trans-esterifying the said racemic alcohol.

6. The process of claim 5, wherein the asymmetric trans-esterification is carried out in the presence of vinyl propionate as an esterification agent.

7. The process of claim 5, wherein the asymmetric trans-esterification is carried out in the presence of lipase derived from Candida antarcia as a catalyst.

8. The process of claim 7, wherein the lipase is immobilized on porous acrylic resin particles.

9. The process of claim 7, wherein the lipase is used in an amount of 0.1 to 10 g per mole of the racemic alcohol.

10. The process of claim 5, wherein the trans-esterification is carried out at a temperature ranging from 20 to 40°C.
